# EUROPEAN PATENT APPLICATION

(11) **EP 1 691 313 A2**
(43) Date of publication of application: **16.08.2006**
(21) Application number: 06002927.9
(22) Date of filing: 14.02.2006
(51) Int. Cl.: G06F 19/00

(54) **Operation information management device and surgery system**

(30) Priority: 14.02.2005 JP 2005036976
(71) Applicant: Olympus Corporation, Shibuya-ku, Tokyo (JP)
(72) Inventor: Ozaki, Takashi, Hachioji-shi Tokyo 192-8512 (JP); Akinobu, Uchikubo, Hachioji-shi Tokyo 192-8512 (JP); Koichi, Tashiro, Hachioji-shi Tokyo 192-8512 (JP); Chie, Imamiya, Hachioji-shi Tokyo 192-8512 (JP); Takeaki, Nakamura, Hachioji-shi Tokyo 192-8512 (JP); Masakazu, Gotanda, Hachioji-shi Tokyo 192-8512 (JP)
(74) Representative: von Hellfeld, Axel

(57) **Abstract**

An operation information management device (22) for managing a use history of multiple medical apparatuses has a management item setting section (S2) for setting management items which classify the use history of the various medical apparatuses, a management item storing section (S4) for storing the use history information of the various medical apparatuses based on the management items as a database, an analysis designating section (301) for designating analysis items based on the use history of the various medical apparatuses, and an analysis information creating section for searching use history information of the multiple medical apparatuses stored by the management item storing section based on the analysis items and for generating and outputting analysis information.

## Description

### BACKGROUND OF THE INVENTION

### 1. Field of the Invention

The present invention relates to an operation information management device and a surgery system which controls multiple medical apparatuses.

### 2. Description of the Related Art

Recently, endoscope surgery systems which perform procedures using endoscopes have become widespread, and the medical apparatuses used are also wide-ranging.

The medical apparatuses used with such an endoscope surgery system provide electrosurgical knife devices, ultrasonic therapeutic apparatuses, pneumoperitoneum devices, and so forth, in addition to the electronic endoscope systems, and as proposed in Japanese Unexamined Patent Application Publication No. 2003-76786 and Japanese Unexamined Patent Application Publication No. 2003-70748, these apparatuses are integrated-managed as a system, and are controlled by a operating apparatus arranged under a system controller.

However, as described above, with the endoscope surgery system, multiple medical apparatuses are used, but the skill of the surgeons, the number of times of the apparatus being used during surgery due to the difficulty of the procedure, and operating content may differ, and also the length of surgery may differ, and so it may be difficult to objectively analyze the procedure after the surgery for example.

Also, the apparatuses and consumable supplies are not managed in real time according to use status, and so maintenance for each apparatus and for each consumable supply is difficult to perform effectively.

The present invention has been made in light of the above situation, and accordingly, it is an object of the present invention to provide an operation information management device and surgery system which can objectively analyze the procedure after surgery easily and appropriately.

### SUMMARY OF THE INVENTION

An operation information management device for managing use history of multiple medical apparatuses of an embodiment of the present invention has a management item setting section for setting management items which classify the use history of the various medical apparatuses, a management item storing section for storing the use history information of the various medical apparatuses based on the management items as a database, an analysis designating section for designating analysis items based on the use history of the various medical apparatuses, and an analysis information creating section for searching for use history information of the multiple medical apparatuses stored by the management item storing section based on the analysis items and for generating and outputting analysis information.

### BRIEF DESCRIPTION OF THE DRAWINGS

Fig. 1 is an overall view illustrating an endoscope surgery system according to an embodiment of the present invention;
Fig. 2 is a diagram describing the primary portions of the light source device in Fig. 1;
Fig. 3 is a diagram showing a hand piece used in the electrosurgical knife device shown in Fig. 1;
Fig. 4 is a diagram showing a placement example of a data storage unit which stores various apparatus information of the endoscope surgery system shown in Fig. 1;
Fig. 5 is a flowchart showing a process during a procedure of the endoscope surgery system shown in Fig. 1;
Fig. 6 is a diagram showing a management item setting screen developed on an operation panel during the process in Fig. 5;
Fig. 7 is a diagram showing an analysis item selection screen developed on the operation panel shown in Fig. 1 for inputting analysis items for the purpose of analysis after the procedure;
Fig. 8 is a flowchart showing the flow of the apparatus output analysis (numerical comparison) processing selected in the analysis item selection screen shown in Fig. 7;
Fig. 9 is a diagram showing an analysis screen developed in the processing in Fig. 8;
Fig. 10 is a flowchart showing the flow of the apparatus output analysis (chronological comparison) processing selected in the analysis item selection screen shown in Fig. 7;
Fig. 11 is a diagram showing an analysis screen developed in the processing in Fig. 10;
Fig. 12 is a diagram showing an error information window which is developed during the error analysis selected in the analysis item selection screen in Fig. 7;
Fig. 13 is a flowchart showing the flow of the analysis by apparatus (all periods) selected in the analysis item selection screen shown in Fig. 7;
Fig. 14 is a diagram showing an analysis screen developed in the processing in Fig. 13;
Fig. 15 is a flowchart showing the flow of the analysis by apparatus (specified period) selected in the analysis item selection screen shown in Fig. 7;
Fig. 16 is a diagram showing an analysis screen developed in the processing in Fig. 15;
Fig. 17 is a diagram showing a remaining consumable supplies information window developed at the time of remaining consumable supplies analysis selected in the analysis item selection screen in Fig. 7; and
Fig. 18 is a diagram showing a modification example of the location of the data storage unit storing the various apparatus information of the endoscope surgery system shown in Fig. 1.

### DESCRIPTION OF THE PREFERRED EMBODIMENTS

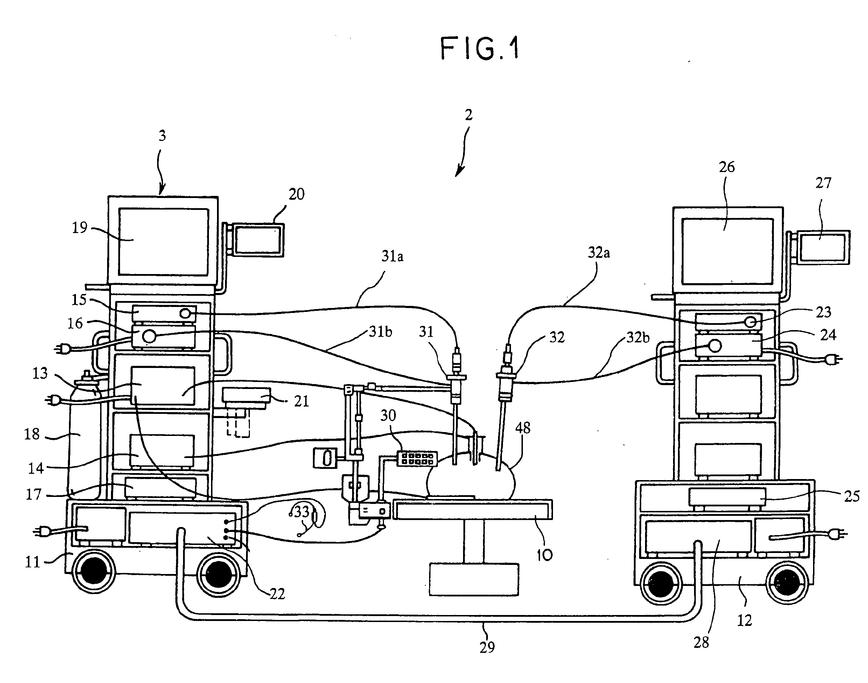

Embodiments of the present invention will be described with reference to the drawings. Figs. 1 through 18 are diagrams relating to an embodiment of the present invention.

First, an overall configuration of an endoscope surgery system 3 which is a medical system located in an operating room 2 will be described with reference to Fig. 1. As shown in Fig. 1, a patient bed 10 whereupon a patient 48 lies, and an endoscope surgery system 3 are located within the operating room 2. The endoscope surgery system 3 has a first cart 11 and a second cart 12.

The first cart 11 has loaded thereupon medical apparatuses which are controlled devices, such as an electrosurgical knife device 13, pneumoperitoneum device 14, an endoscope camera device 15, a light source device 16, and a video tape recorder (VTR) 17, and a gas cylinder 18 filled with carbon dioxide. The endoscope camera device 15 is connected to a first endoscope 31 via a camera cable 31a. The light source device 16 is connected to the first endoscope 31 via a light guide cable 31b.

Also, the first cart 11 has loaded thereupon a display device 19, a first intensive display panel 20, and an operation panel 21. The display device 19 is for example a TV monitor which displays the endoscope images and the like.

The intensive display panel 20 is a display means which can selectively display various data during the surgery. The operation panel 21 is configured for example with a display unit such as a liquid crystal display and for example an integrated touch panel provided on this display unit, and the nurses in the non-sterilized area operate this central operation device. In other words, the operation panel 21 comprises the display device.

Further, a system controller 22 which is a control device is also placed on the first cart 11. This system controller 22 is connected to the above-described electrosurgical knife device 13, pneumoperitoneum device 14, endoscope camera device 15, light source device 16, and VTR 17 via a communication line (not shown). The system controller 22 can be connected to a headset-type microphone 33, and the system controller 22 can recognize the vocal sound input from the microphone 33, and the various apparatuses can be controlled by the voice of the surgeon.

On the other hand, an endoscope camera device 23 which is a controlled device, a light source device 24, an image processing device 25, a display device 26, and a second intensive display panel 27 are placed on the second cart 12.

The endoscope camera device 23 is connected to a second endoscope 32 via a camera cable 32a. The light source device 24 is connected to the second endoscope 32, via a light guide cable 32b.

The display device 26 displays the endoscope images and so forth that are captured on the endoscope camera deice 23. The second intensive display panel 27 can selectively display various data during surgery.

The endoscope camera device 23, light source device 24 and image processing device 25 are connected to a relay unit 28 placed on the second cart 12 via a communication wire not shown. Also, the relay unit 28 is connected to the system controller 22 placed on the above-described first cart 11, via a relay cable 29.

Thus, the system controller 22 centrally controls the endoscope camera device 23, light source device 24, and image processing device 25 on the second cart 12, and the electrosurgical knife device 13, the pneumoperitoneum device 14, the endoscope camera device 15, the light source device 16, and the VTR 17 on the first cart 11. Thus, in the case that communication is performed between the system controller 22 and these devices, the system controller 22 can display setting screens such as the setting status of the connected devices or the operation switches and so forth, on the liquid crystal display on the operation panel 21. Further, the system controller 22 can have operation input performed such as changes to the setting values, by desired operation switches of the operation panel 21 being touched and the touch panel of the specified region being operated.

A remote controller 30 is a second central operating device to be operated by surgeons in a sterilized area, and the other devices which are in communication can be operated via the system controller 22.

The system controller 22 is connected to a patient monitoring system (not shown), and the biologic information acquired from the patient monitoring system is analyzed, and the analysis result can be displayed on a specified display device.

Also, the system controller 22 has an infrared communication port (not shown) which is a communication means affixed. The infrared communication port is provided in a location such as the vicinity of the display device 19 where the infrared ray can be easily cast, and is connected to the system controller 22 with a cable.

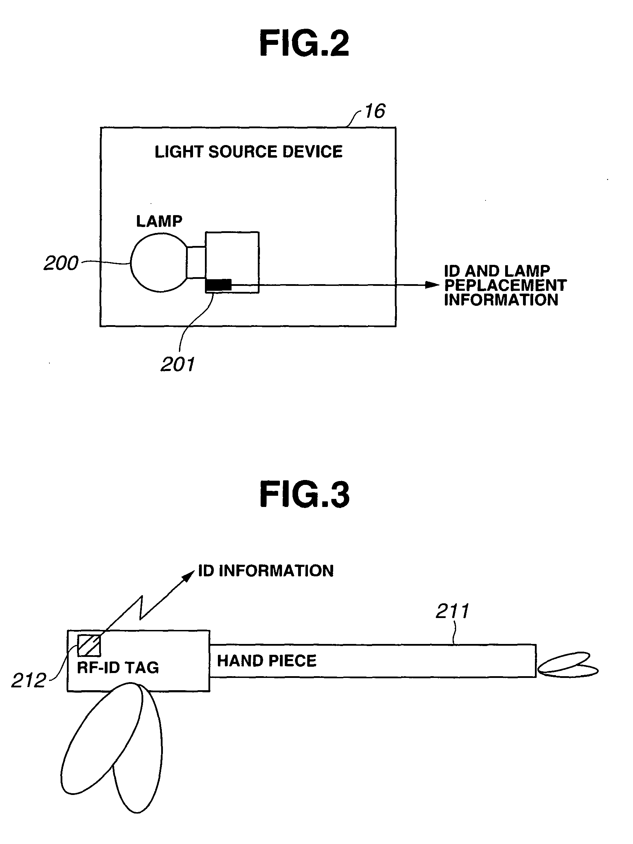

For example, the light source device 16 of the endoscope surgery system 9 can have a lamp 200 replaced, as shown in Fig. 2, and when the lamp 200 is replaced, the ID of the light source device 16 and the lamp replacement information are output from a reset unit 201 to the system controller 22. Thus with the system controller 22, the information of whether the light source device 16 is lit or not lit, and the lamp replacement information can be managed in real time.

Also, for example a hand piece 211 used in the electrosurgical knife device 13 has an RF-ID tag 212 provided, as shown in Fig. 3, and the ID of the hand piece 211 is output to the system controller 22 by the RD-ID reading device not shown. Thus, with the system controller 22, the hand piece can be managed in real time with the ID for each hand piece 211.

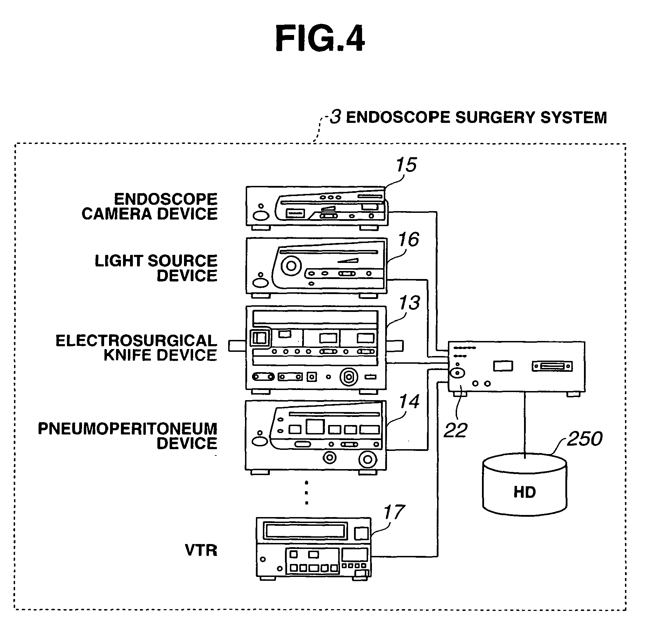

As illustrated in Fig. 4, the various apparatuses of the endoscope surgery system 3 such as the electrosurgical knife device 13, pneumoperitoneum device 14, endoscope camera device 15, light source device 16, and VTR 17 are controlled by the system controller 22, and the use history information of the various apparatuses are placed in a database by the system controller 22, and is stored in a data storage unit 250 which is a storage device such as a hard disk, and managed. The system controller 22 comprises the operation information management device which manages the use history of the multiple medical apparatuses.

The operation of the present embodiment thus configured will be described.

When a procedure starts, the system controller 22 obtains the surgery information such as the procedure name, name of surgeon, and date of surgery performed, which is input with the operation panel 21 in Step S1.

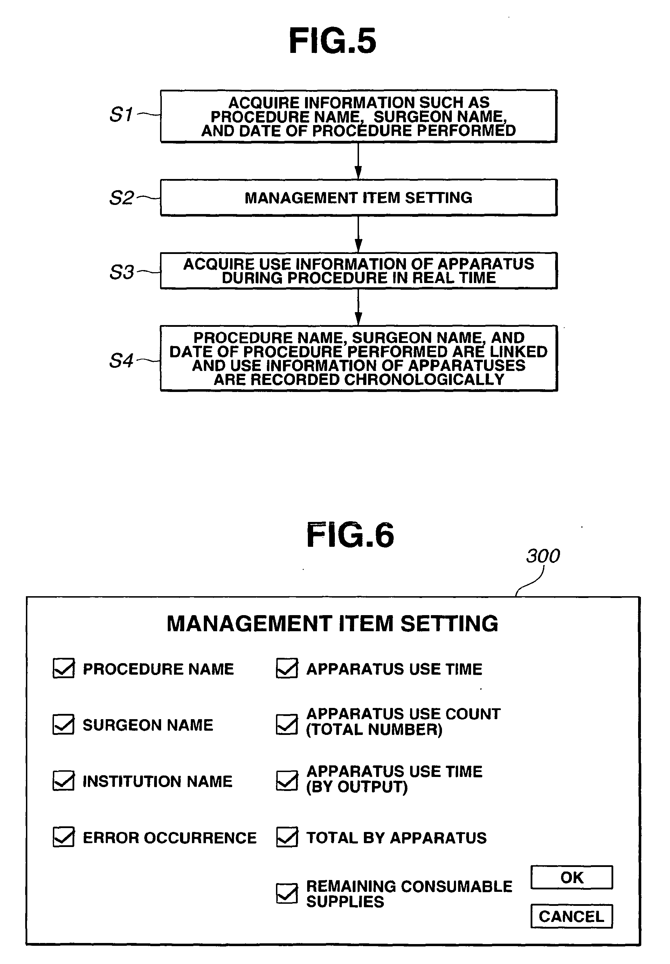

In Step S2, a management item setting screen 300 is displayed on the operation panel 21 such as that shown in Fig. 6, and the system controller 22 performs management item setting for use history management, by the nurses touching the touch panel and checking each management item displayed on the display unit. Step S2 comprises the management item setting section for setting the management items which classifies the use history of the various medical apparatuses. Fig. 6 shows procedure name, surgeon name, institution name, error occurrence, apparatus use time, apparatus use count (total number), apparatus use time (by output), total by apparatus, and remaining consumable supplies, as examples of management items.

During surgery, use information including output value of the apparatus during the procedure is acquired in real time in Step S3, and is linked to the management items set on the management item setting screen 300 in Step S4, and the use information of the apparatuses is recorded on the data recording unit 250 as chronological apparatus use history. Step S4 comprises the management item storing section which places the use history information of the various medical apparatuses based on the management items into a database and stores this.

By thus using the use history of the apparatuses recorded on the data recording unit 250, various types of analysis can be done after the procedure with the system controller 22.

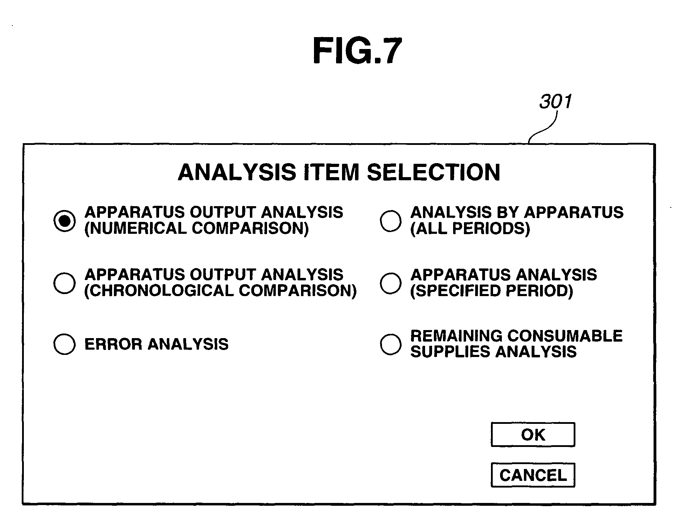

With the system controller 22, by displaying an analysis item selection screen 301 on the operation panel 21, such as that shown in Fig. 7, input for the analysis items to execute procedure analysis is awaited for apparatus output analysis (numerical comparison), apparatus output analysis (chronological comparison), error analysis, analysis by apparatus (all periods), apparatus analysis (specified period), and remaining consumable supplies analysis. An analysis item selection screen 301 such as that shown in Fig. 7 is displayed with the system controller 22, and the processing for inputting and designating the analysis item to the analyst such as nurses who analyze the procedure comprises the analysis designating section which designates the analysis item based on the use history of the various medical apparatuses.

Hereafter, the processing with the system controller 22 corresponding to the analysis items will be described. The various analysis processing with the system controller 22 to be described below comprises the analysis information generating section which searches for use history information of the aforementioned multiple medical apparatuses stored in the data recording unit 250 based on the analysis items, creates analysis information, and outputs this.

### (1) Apparatus output analysis (numerical comparison)

When the apparatus output analysis (numerical comparison) item is selected in the analysis item selection screen 301, the system controller 22 acquires the necessary information by the nurses operating the touch panel in Step S11 and inputting information such as procedure name, surgeon name, and data of surgery performed from the operating panel 21. Next, the controller 22 reads the use information of the apparatus with the data recording unit 250 in Step S12, based on the information such as procedure name, surgeon name, and data of surgery performed.

Then the system controller 22 acquires the necessary information by the nurses operating the touch panel in Step S13 and inputting the apparatus name (apparatus ID) from the operating panel 21. Then, the system controller 22 reads all use information of the apparatus with the same procedures, from the data recording unit 250, based on the apparatus name (apparatus ID). Here, the output value and the output count of the apparatus are read.

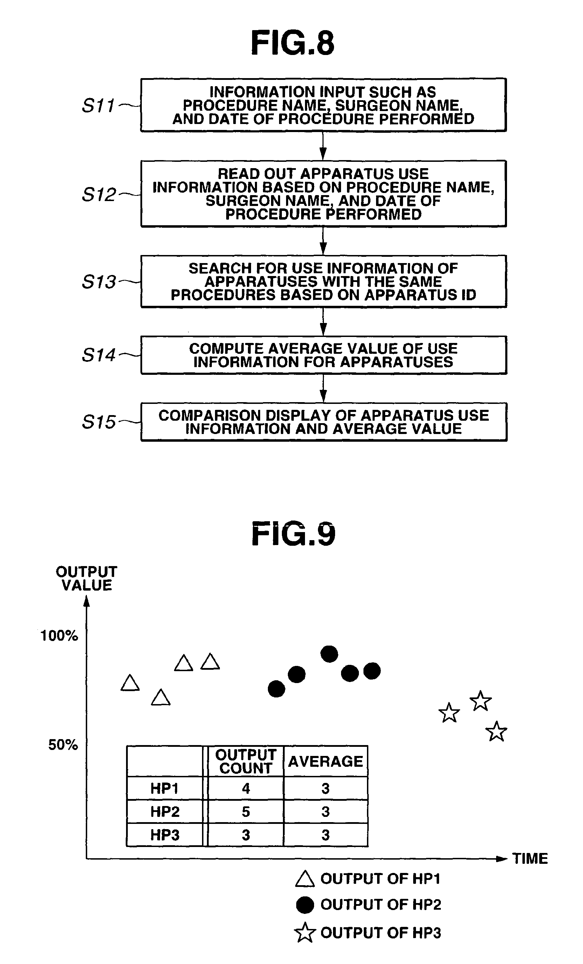

Next, the system controller 22 computes and creates the average value of all use information of apparatuses with similar types of procedures read out in Step S14. The system controller 22 displays the use information and average values of the apparatuses as display information in Step S15 so as to make comparisons with the operation panel 21 as shown in Fig. 9.

Fig. 9 shows an example of a display that can compare a graph for chronological use status (timing and output value) of each hand piece and a chart for the average value of the past use performance (here, average value of output count) in the event that an electrosurgical knife for a procedure uses multiple, for example 3 types of hand pieces HP1, HP2, and HP3. With this display, for example, the apparatus use status for the current procedure and the past performance can be compared qualitatively and quantitatively, and the degree of difficulty of the current procedure, or the skill level of the surgeon can be determined objectively.

### (2) Apparatus output analysis (chronological comparison)

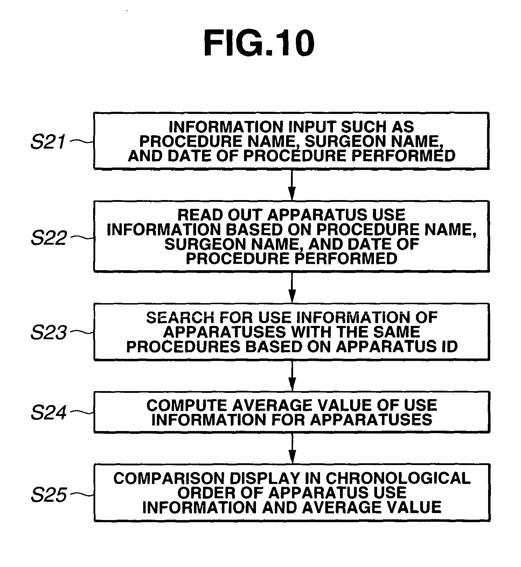

When the apparatus output analysis (chronological comparison) item is selected in the analysis item selection screen 301, the system controller 22, as shown in Fig. 10, acquires the necessary information by the nurses operating the touch panel in Step S21 and inputting information such as procedure name, surgeon name, and data of surgery performed from the operating panel 21. Next, the controller 22 reads the use information of the apparatus with the data recording unit 250 in Step S22, based on the information such as procedure name, surgeon name, and data of surgery performed.

Then the system controller 22 acquires the necessary information by the nurses operating the touch panel in Step S23 and inputting the apparatus name (apparatus ID) from the operating panel 21, based on the apparatus name (apparatus ID), the system controller 22 reads all use information of the apparatus with the same procedures, that is to say, all output information, from the data recording unit 250.

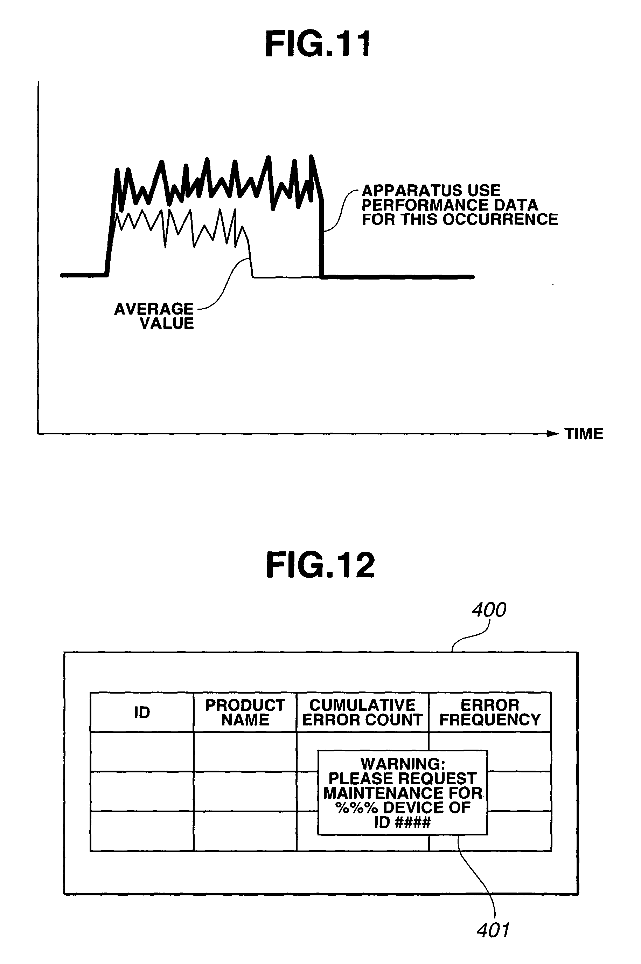

Next, the system controller 22 computes the use trends for the apparatuses with the same procedures where are read out, that is to say, computes the average value of the chronological output value in Step S24. In step S25, the system controller 22 displays a comparison of the chronological change and chronological average of the output value for apparatuses with the current procedure, as display information to the operation panel 21 as shown in Fig. 11. With this display, for example, the apparatus use status for the current procedure and the average of the past performance can be compared qualitatively and quantitatively, and the degree of difficulty of the current procedure, or the skill level of the surgeon can be determined objectively.

### (3) Error Analysis

When the error analysis item is selected in the analysis item selection screen 301, the system controller 22 acquires the necessary information by the nurses operating the touch panel and inputting the apparatus name (apparatus ID) from the operating panel 21. The system controller 22 extracts past error information for the apparatus which is the apparatus name (apparatus ID) from the data recording unit 250, computes the cumulative error count and error frequency for each apparatus, outputs this as display information, and displays an error information window 400 in the operation panel 21 as shown in Fig. 12.

The system controller 22 manages a cumulative error count and an error frequency with an apparatus ID, and for example if the frequency of error generating is too high (for example, in the case that the cumulative error count or the error frequency reaches the predetermined threshold), a warning information is created and output in order to have a warning display such as a pop-up window 401 on top of the error information window 400.

### (4) Analysis by apparatus (All periods)

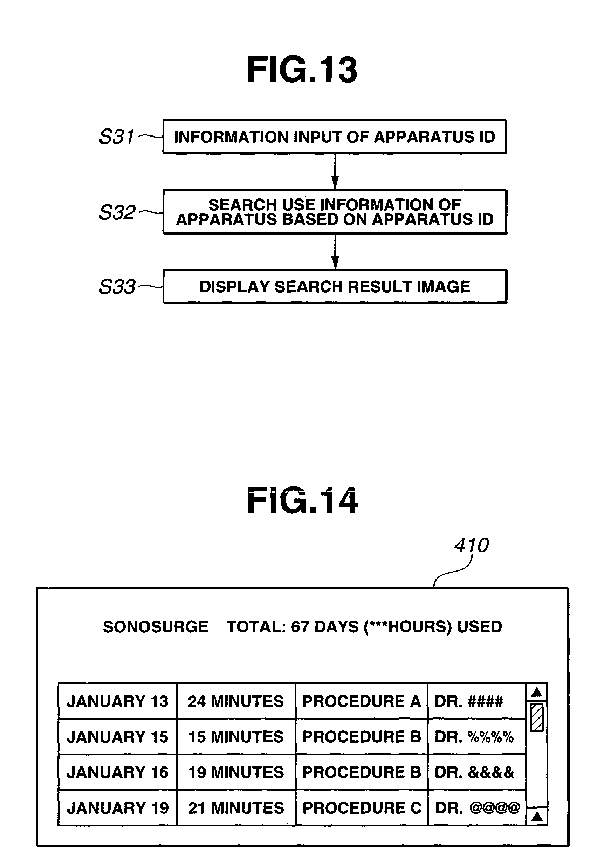

When the analysis by apparatus (all periods) item is selected in the analysis item selection screen 301, the system controller 22, as shown in Fig. 13, acquires the necessary information by the nurses operating the touch panel in Step S31 and by inputting and designating the apparatus name (apparatus ID) from the operating panel 21. The system controller 22 searches for past use performance information of the apparatus regarding which the apparatus name (apparatus ID) is designated by the recording unit 250 in Step S32, and computes the total use time. The system controller 22 displays an all-periods use performance window 410 in the operation panel 21 as search result images as shown in Fig. 14 in Step S33. With the windows 410 in Fig. 14, display information is displayed in the form of a chart, for example, the apparatus to be considered is used at what time, during how much time, by which procedure, by which doctor, and so forth.

In Fig. 14 shown an example of the all-period use performance window 410 of for example an ultrasonic operating device (labeled as sonosurge in Fig. 14) for each apparatus, and can analyze how much time is used for which apparatus, and how it is used most often.

### (5) Apparatus analysis (Specified period)

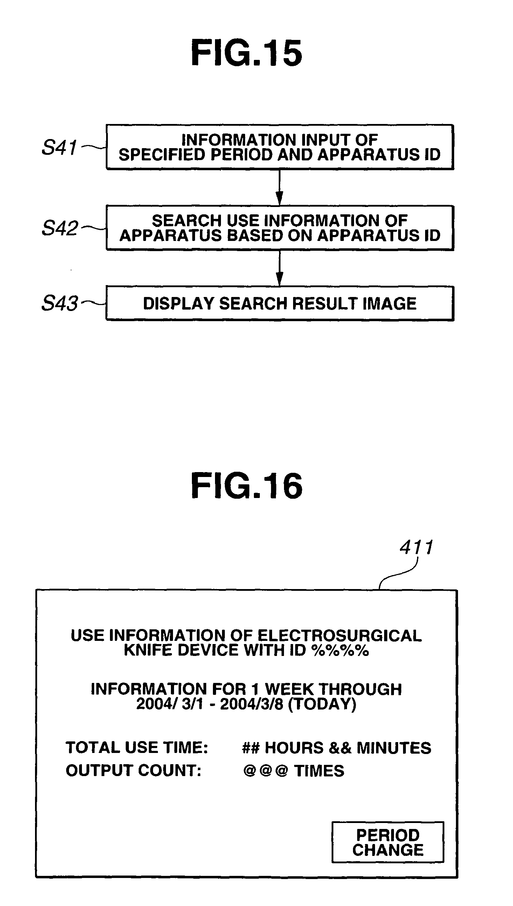

When the analysis by apparatus (specified periods) item is selected in the analysis item selection screen 301, the system controller 22, as shown in Fig. 15, acquires the necessary information by the nurses operating the touch panel and by inputting and designating the specified period which is the detecting period, and the apparatus name (apparatus ID) from the operating panel 21 in Step S41. The system controller 22 searches for past detecting period use performance information of the apparatus regarding which the apparatus name (apparatus ID) is designated by the recording unit 250 in Step S42, and computes the total use time. The system controller 22 displays a specified period use performance window 411 in the operation panel 21 as a search result image as shown in Fig. 16, as display information in Step S43. The window 411 in Fig. 16 displays how much time (in other words cumulative total use time) is used during the specified period by the apparatus, and the count of the output number of times.

With Fig. 16 shows an example of the specified period use performance window 411 for the electrosurgical knife for example for each apparatus, and by displaying the use information for the specified period for each apparatus, the management of the apparatus such as maintenance can be appropriately performed. Also by selecting the period change button, the period for use information can be set at will.

### (6) Remaining Consumable Supplies Analysis

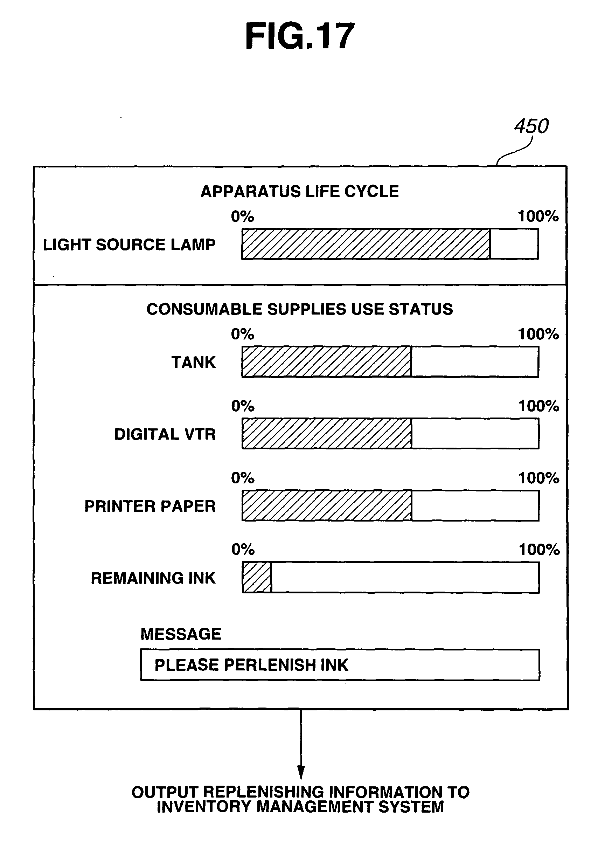

When the remaining consumable supplies analysis is selected in the analysis item selection screen 301, the system controller 22 searches the status information of the consumable supplies of the apparatuses connected to the system from the data recording unit 250, and as display information displays a consumable supplies information window 450 as shown in Fig. 17 on the operation panel 21. With the consumable supplies information window 450 in Fig. 17, for each consumable supply, the use cumulative time, use amount, remaining amount and so forth are displayed as use status, and additionally, for consumable supplies with a use status which have surpassed the predetermined threshold also displays a message output with instructions for replenishing. The consumable supplies information window 450 can be output to an inventory management system (not shown) with replenishing information by the operation panel 21, and the use status of the consumable supplies can be analyzed and appropriate inventory management can be performed.

With the surgery system of the present embodiment, the use history of the apparatuses is placed in a database with the management items as a standard, and by specifying the analysis items, the use history in a database is searched, and the use history of the apparatus is analyzed based on the analysis items. As a result, the analyst such as the surgeon or the manager can objectively analyze the procedures easily and appropriately after the surgery, and also maintenance for each apparatus and consumable supply can be performed efficiently.

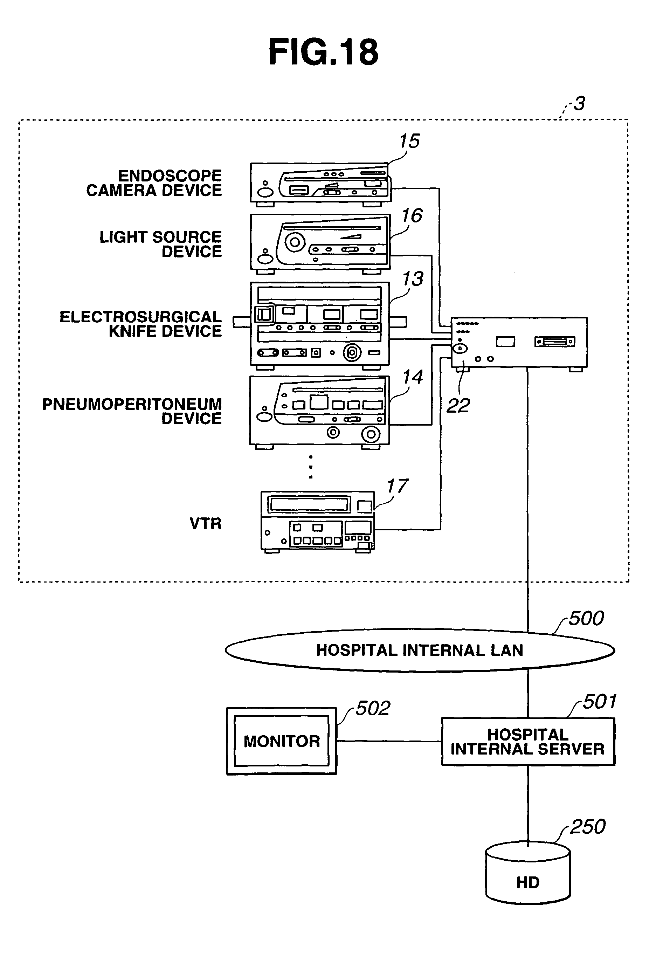

With the present embodiment, various types of apparatus information is stored in the data recording unit 250 under the control of the system controller 22, but should not be limited to this, and for example as shown in Fig. 18, the system controller 22 can be connected to a hospital internal server 501 via a hospital internal LAN 500, and the various types of apparatus information can be stored in the data recording unit 250 under the control of the hospital internal server 501, and the above analysis can be displayed on a monitor 502.

Thus, according to the embodiments of the present invention, the advantage is that the procedures can be objectively analyzed after surgery, easily and appropriately.

The present invention is not limited to the above-described embodiments, and various changes and modifications can be made without departing from the spirit or scope of the invention.

## Claims

1. An operation information management device for managing a use history of multiple medical apparatuses, said device comprising:
a management item setting section for setting management items which classify the use history of the various medical apparatuses;
a management item storing section for storing the use history information of the various medical apparatuses based on the management items, as a database;
an analysis designating section for designating analysis items based on the use history of the various medical apparatuses; and
an analysis information creating section for searching for use history information of the multiple medical apparatuses stored by the management item storing section based on the analysis items, and for generating and outputting analysis information.

2. The operation information management device according to Claim 1, wherein; the analysis designating section includes one of the output value comparison of the various medical apparatuses, error analysis, use time, and remaining consumable supplies analysis as analysis items.

3. The operation information management device according to Claim 1, wherein the management items include items for output value and output count of the various medical apparatuses, and the analysis information creating section creates and outputs the output value and output count of the medical apparatuses designated for the various surgeries, as display information for the display devices.

4. The operation information management device according to Claim 2, wherein the analysis information creating section computes, creates, and outputs, the average value of the output count for the same procedures with the designated medical apparatuses, as display information.

5. The operation information management device according to Claim 1, wherein the management items include an item for output value of the various medical apparatuses, and the analysis information creating section creates and outputs the chronological output value data of the medical apparatuses designated for surgery, as display information for the display devices.

6. The operation information management device according to Claim 5, wherein the analysis information creating section computes the average value of the output value of various medical apparatuses in the same procedures for the designated medical apparatuses as display information, and creates and outputs this as chronological average value data.

7. The operation information management device according to Claim 1, wherein the management items include an item for errors generated by the various medical apparatuses, and the analysis information creating section creates and outputs a cumulative number or frequency of errors of the various medical apparatuses, as display information for the display devices.

8. The operation information management device according to Claim 7, wherein the analysis information creating section creates and outputs warning information as display information, when the cumulative number or frequency of errors reach a specified threshold.

9. The operation information management device according to Claim 1, wherein the management items include an item for use time of the various medical apparatuses, and the analysis information creating section computes the total use time over all periods for the designated medical apparatuses, and creates and outputs this as display information for the display devices.

10. The operation information management device according to Claim 9, wherein the management items further include an item for the procedure, and the analysis information creating section creates the total use time for each procedure, and outputs this as display information.

11. The operation information management device according to Claim 1, wherein the management items include an item for use time of the various medical apparatuses, and the analysis information creating section computes the total use time over a specified period for the designated medical apparatuses, and creates and outputs this as display information for the display devices.

12. The operation information management device according to Claim 11, wherein the management items further include an item for output count of the various medical apparatuses, and the analysis information creating section computes the output count during a specified period, and creates and outputs this as display information.

13. The operation information management device according to Claim 1, wherein the management items further include an item for use status of consumable supplies for the various medical apparatuses.

14. The operation information management device according to Claim 13, wherein the use status is the use time or use amount of the consumable supplies, and the analysis information creating section creates and outputs replenishing information as display information, based on the use time or use amount.

15. A surgery system, comprising:
multiple medical apparatuses; and
a medical information management device connected to the multiple medical apparatuses, the medical information management device further comprising:
a management item setting section for setting management items which classify the use history of the various medical apparatuses;
a management item storing section for storing the use history information of the various medical apparatuses based on the management items, as a database;
an analysis designating section for designating analysis items based on the use history of the various medical apparatuses; and
an analysis information creating section for searching for use history information of the multiple medical apparatuses stored by the management item storing section based on the analysis items, and for generating and outputting analysis information.

16. The surgery system according to Claim 15, wherein the analysis designating section includes one of the output value comparison of the various medical apparatuses, error analysis, use time, and remaining consumable supplies analysis as analysis items.

17. The surgery system according to Claim 15, wherein the management items include items for output value and output count of the various medical apparatuses, and the analysis information creating section creates and outputs the output value and output count of the medical apparatuses designated for the various surgeries, as display information for the display devices.

18. The surgery system according to Claim 15, wherein; the management items include an item for output value of the various medical apparatuses, and the analysis information creating section creates and outputs the chronological output value data of the medical apparatuses designated for surgery, as display information for the display devices.

19. The surgery system according to Claim 15, wherein the management items include an item for errors generated by the various medical apparatuses, and the analysis information creating section creates and outputs a cumulative number or frequency of errors of the various medical apparatuses, as display information for the display devices.

20. The surgery system according to Claim 15, wherein the management items include an item for use time of the various medical apparatuses, and the analysis information creating section computes the total use time over all periods or a specified period for the designated medical apparatuses, and creates and outputs this as display information for the display devices.
